(19) Europäisches Patentamt

European Patent Office

Office européen des brevets

(11) Veröffentlichungsnummer: **0 464 554 B1**

(12) **EUROPÄISCHE PATENTSCHRIFT**

(45) Veröffentlichungstag der Patentschrift: **30.08.95**

(21) Anmeldenummer: **91110369.5**

(22) Anmeldetag: **24.06.91**

Die Akte enthält technische Angaben, die nach
dem Eingang der Anmeldung eingereicht wurden
und die nicht in dieser Patentschrift enthalten sind.

(51) Int. Cl.⁶: **G01N 33/563**, G01N 33/546,
G01N 33/547, G01N 33/68

(54) **Durchführung eines homogenen Immunoassays nach dem Agglutinationsprinzip.**

(30) Priorität: **25.06.90 DE 4020204**

(43) Veröffentlichungstag der Anmeldung:
**08.01.92 Patentblatt 92/02**

(45) Bekanntmachung des Hinweises auf die
Patenterteilung:
**30.08.95 Patentblatt 95/35**

(84) Benannte Vertragsstaaten:
**AT BE CH DE DK ES FR GB GR IT LI LU NL SE**

(56) Entgegenhaltungen:
**EP-A- 0 307 754**
**EP-A- 0 331 068**
**EP-A- 0 349 988**
**EP-A- 0 356 964**

(73) Patentinhaber: **BOEHRINGER MANNHEIM
GMBH**

**D-68298 Mannheim (DE)**

(72) Erfinder: **Deger, Arno, Dr.
Föhrenstrasse 3
W-8124 Seeshaupt (DE)**
Erfinder: **Guillot, François, Dr.
Rue Cheneviere, 21
F-38240 Meylan (FR)**
Erfinder: **Berger, Michael, Dr.
Knappenstrasse 16
W-8122 Penzberg (DE)**

(74) Vertreter: **Huber, Bernhard, Dipl.-Chem. et al
Patentanwälte
H. Weickmann, Dr. K. Fincke
F.A. Weickmann, B. Huber
Dr. H. Liska, Dr. J. Prechtel, Dr. B.
Böhm
Postfach 86 08 20
D-81635 München (DE)**

## Beschreibung

Die Erfindung betrifft ein Verfahren zur Durchführung eines homogenen Immunoassays nach dem Agglutinationsprinzip unter Verwendung eines Konjugats aus einem Fab'-Fragment und einer Komponente K.

In Körperflüssigkeiten und Geweben kommen sehr viele Substanzen vor, die mit einem spezifischen Bindungspartner bindefähig sind und als Parameter für bestimmte Erkrankungen oder den Gesundheitszustand des menschlichen Körpers dienen. Hierzu zählen einerseits immunologisch aktive Proteine, die Bindungsstellen an ihrer Oberfläche aufweisen wie z.B. Tumormarker, Hormone oder virale Proteine und andererseits DNA-Fragmente. Da diese Substanzen oft nur in sehr geringen Mengen vorkommen, verwendet man zu ihrem Nachweis Verfahren nach dem Prinzip des Immunoassays, mit denen diese Substanzen sehr spezifisch und genau bestimmt werden können. Dazu gibt es viele Varianten. Die verschiedenen immunologischen Bestimmungsverfahren lassen sich in homogene und heterogene Verfahren einteilen. Bei dem heterogenen Verfahren ist immer eine Festphasen-Reaktion beteiligt, um Komplexe, die die nachzuweisende Substanz und eine markierte Komponente enthalten, zu immobilisieren und dadurch von ungebundenen Komponenten abzutrennen. Bei der homogenen Verfahrensvariante erfolgt keine Trennung von gebundener Markierung und ungebundener Markierung, so daß eine Differenzierung von gebundener und ungebundener Markierung durch andere Methoden erfolgen muß.

Hierzu gibt es verschiedene Möglichkeiten.

Aus DE-OS 27 49 956 ist ein Verfahren zum Nachweis von Proteinen bekannt, das auf der Auswertung einer Agglutinationsreaktion beruht. Dabei werden Antikörper gegen die zu bestimmende Substanz direkt an die agglutinierbaren Teilchen gebunden. Durch diese Bindung kann jedoch die Reaktivität der Antikörper beeinträchtigt werden. Außerdem ist ein derartiges Bestimmungsverfahren anfällig für Störungen durch Rheumafaktoren.

In EP-A 0 356 964 wird ein homogenes Nachweisverfahren beschrieben, das bereits die Nachteile der bis dahin bekannten Verfahren überwindet. Dabei wird die Probelösung mit mindestens zwei Rezeptoren inkubiert, die miteinander bindefähig sind und von denen einer mit der nachzuweisenden Substanz binden kann. Dabei kann eine Agglutination nur erfolgen, wenn die nachzuweisende Substanz mit beiden Rezeptoren bindet, nicht jedoch, wenn die Rezeptoren miteinander binden oder die nachzuweisende Substanz nur mit einem der beiden Rezeptoren bindet. Als mit der nachzuweisenden Substanz bindefähige Rezeptoren werden Konjugate eingesetzt aus einem Partner eines spezifisch miteinander bindenden Paares und einer mit der zu bestimmenden Substanz spezifisch bindefähigen Komponente K. Wird das hier beschriebene Prinzip zum Nachweis von Proteinen, die mehrere gleiche Epitope aufweisen, eingesetzt, so ist als mit der nachzuweisenden Substanz bindefähiger Rezeptor ein Konjugat einzusetzen, das nur eine einzige Bindestelle für die nachzuweisende Substanz aufweist, um eine unspezifische Agglutination auszuschließen.

EP-A-0 307 754 beschreibt die Herstellung von papainfreien $F(ab')_2$-Antikörperfragmenten, die aus einem mit Papain gespaltenen vollständigen IgG-Antikörper erhalten werden. Aus den $F(ab')_2$-Antikörperfragmenten können Fab'-Antikörperfragmente mit freien Sulfhydrylgruppen in der Hinge-Region erhalten werden.

Aufgabe der vorliegenden Erfindung war es, ein Verfahren zur Durchführung homogener Immunoassays zur Verfügung zu stellen, bei dem unspezifische Agglutinationen möglichst vermieden werden, so daß genaue und reproduzierbare Ergebnisse erhalten werden.

Diese Aufgabe wird gelöst durch ein Verfahren zur Durchführung eines homogenen Immunoassays nach dem Agglutinationsprinzip unter Verwendung eines Konjugats aus einem Fab'-Fragment und einer Komponente K, das dadurch gekennzeichnet ist, daß man ein Konjugat verwendet, welches erhalten wird indem man ein $F(ab')_2$-Fragment eines Antikörpers der Immunglobulinklasse G reduzierenden Bedingungen aussetzt und anschließend mit der Komponente K, die entweder eine zur Bindung geeignete funktionelle Gruppe aufweist oder durch Einführung einer funktionellen Gruppe in geeigneter Weise derivatisiert wurde, umsetzt, wobei die Komponente K über die funktionelle Gruppe an die bei der Reduktion entstandenen freien SH-Gruppen des Fab'-Fragmentes gebunden wird und dazu agglutinierbare Teilchen verwendet, welche eine mit K bindefähige Substanz tragen.

Überraschenderweise wurde gefunden, daß bei Verwendung dieser Konjugate unspezifische Agglutinationen in hohem Maße vermieden werden können. Durch die Verwendung der monovalenten Bindepartner konnten Verfahren nach dem Prinzip des Agglutinations-Immunoassay in ihrer Genauigkeit und Reproduzierbarkeit weiter verbessert werden.

Für das erfindungsgemäße Verfahren werden Konjugate aus einem Fab'-Fragment eines IgG-Antikörpers, der mit der nachzuweisenden Substanz spezifisch bindefähig ist und einer Komponente K verwendet. Derartige Konjugate können hergestellt werden, indem man die jeweils einzusetzenden Antikörper in an sich bekannter Weise mit Pepsin behandelt. Dabei entstehen als F(ab')$_2$ bezeichnete Fragmente, die zwei Paratope aufweisen und bei denen die zwei schweren Ketten durch Disulfidbindungen zusammengehalten werden. Die F(ab')$_2$-Fragmente werden dann milden reduzierenden Bedingungen ausgesetzt, so daß bevorzugt die intramolekularen Disulfide in der Hinge-Region getrennt werden, nicht jedoch die Disulfidbrükken zwischen leichter und schwerer Kette. Bevorzugt wird daher die Reduktion mit einem milden Reagenz durchgeführt. Geeignet sind hierzu z.B. Cysteamin, Cystein, Mercaptoethanol oder Borhydrid. Auf diese Weise werden Fab'-Fragmente erhalten, die 1 bis 3 freie SH-Gruppen aufweisen. Alle freien SH-Gruppen stehen für eine Bindung mit der verwendeten Komponente zur Verfügung. Es hat sich jedoch herausgestellt, daß aufgrund sterischer Hinderung entweder nur eine Komponente gebunden wird oder aber von zwei oder mehreren gebundenen Komponenten nur eine ihre Aktivität behält.

An diese Fab'-Fragmente wird eine Komponente K gebunden, die ein Partner eines spezifisch bindenden Paares ist. Die Komponente K kann nun an das Fab'-Fragment entweder über eine funktionelle Gruppe, die bereits vorhanden ist oder eingeführt wird und die gegebenenfalls aktiviert wird, gebunden werden. Die Bindung erfolgt entweder direkt zwischen SH-Gruppen des Fab'-Fragmentes und funktioneller Gruppe der Komponente K oder aber über einen Spacer.

Die Bindung von Fab'-Fragment und Komponente K kann direkt über die jeweils vorhandenen funktionellen Gruppen erfolgen. In dieser Ausführungsform ist der Anteil unspezifischer Bindungen besonders gering. Ebenso möglich ist es, zur Bindung einen Spacer zu verwenden. Die Länge des Spacers richtet sich dabei nach der Lage der freien SH-Gruppen, die wiederum abhängig ist von der Subklasse des verwendeten IgG-Antikörpers. Es wurde gefunden, daß bis zu 3 Komponenten-Moleküle gebunden werden können, daß aber aufgrund sterischer Hinderung nur eines mit dem Partner des spezifischen Bindepaares bindefähig ist.

Als Spacer sind bifunktionelle Verbindungen geeignet, die eine funktionelle Gruppe aufweisen, die mit der SH-Gruppe bindefähig ist und eine zweite funktionelle Gruppe aufweisen, die gleich oder verschieden sein kann, die mit der Komponente eine kovalente Bindung eingehen kann. Die Länge des Spacers richtet sich nach der Lage der SH-Bindungen, d.h. also nach der Subklasse des Antikörpers, von dem das Fragment stammt. Wenn der Spacer zu lang ist, sorgt er dafür, daß selbst dann, wenn mehrere SH-Gruppen relativ nah beieinander liegen, eine Bindung an alle Komponenten möglich ist, da die sterische Hinderung überwunden wird. Die Länge des Spacers wird daher abhängig von der Lage der SH-Bindungen und damit vom zu verwendenden Fab'-Fragment bestimmt und liegt für Fab'-Fragmente der IgG-Subklasse I im Bereich von 4 bis 16 Atomen, bevorzugt 4 bis 8 Atomen und für Fab'-Fragmente von Antikörpern der Subklasse IgG II im Bereich von 4 bis 10 Atomen, bevorzugt 4 bis 6 Atomen. Bevorzugt wird als Spacer für die Herstellung von Konjugaten aus einer Komponente K und einem Fab'-Fragment eines IgG-Antikörpers der Subklasse I Maleimidobutyllysin und für Konjugate, die Fab'-Fragmente der IgG-Subklasse II enthalten, Maleimidoethylamin verwendet.

Das Konjugat aus Fab'-Fragment und Komponente K wird als Rezeptor bei der Durchführung des homogenen Immunoassays in an sich bekannter Weise eingesetzt. Als weiterer Rezeptor werden bei dem erfindungsgemäßen Verfahren agglutinierbare Teilchen verwendet, welche eine mit der Komponente K bindefähige Substanz tragen. In einer bevorzugten Ausführungsform werden agglutinierbare Teilchen verwendet, an die eine mit der Komponente K bindefähige Substanz über einen bifunktionellen photoreaktiven Spacer kovalent gebunden ist. Derartige agglutinierbare Teilchen können hergestellt werden, indem die mit der Komponente K bindefähige Substanz zunächst mit einem heterobifunktionellen Reagenz, das auf einer Seite eine Arylazidgruppe und auf der anderen Seite eine mit Aminogruppen des Bindepartners reaktive funktionelle Gruppe aufweist, umgesetzt wird. Dabei reagiert diese mit Aminogruppen reaktive Gruppe mit der bindefähigen Substanz. Nach Entfernung von nicht umgesetztem Reagenz wird das biologische Material über einen vorbestimmten Zeitraum mit dem inerten Trägermaterial, das z.B. ein Polystyrollatex mit einer Teilchengröße von unter 100 nm sein kann, zusammengebracht und dann durch Lichteinwirkung die Photoreaktion ausgelöst, wobei über die Bildung eines reaktiven Nitrens aus der Arylazidgruppe die Bindung an den inerten Träger erfolgt. Nicht kovalent gebundenes Material wird anschließend durch Detergentien-Waschungen effektiv abgelöst und über Zentrifugationsverfahren oder Dialyse an Membranen entfernt. Die Oberflächeneigenschaften derartiger Teilchen können gezielt modifiziert werden, indem z.B. ein immunologisch inertes Protein wie z.B. RSA durch eine Vor-, Co- oder Nachbeladung auf die Partikel aufgebracht wird. Dadurch wird auch die Stabilität des Reagenzes erhöht. Der Anteil an spezifisch bindefähiger Substanz pro Partikel ist über die eingesetzte Proteinmenge sowie die Latexkonzentration bei der Beladung definiert und reproduzierbar einstellbar. Weiterhin kann eine Oberfläche mit definierter spezifischer Bindefähigkeit durch Auswahl des heterobifunktionellen Reagenzes bzw. dessen Kettenlänge gesteuert werden. Vorteil dieses Verfahrens ist es, daß man die spezifisch bindefähige Substanz an jede Art von Festphase binden kann, d.h. nicht auf Festphasenteilchen mit funktionellen Gruppen angewiesen ist. Geeignet als Festphase sind insbesondere Homo- und Copolymere von Styrol und Methylstyrol, von Acrylsäure und ihren Estern, von Methacrylsäure und ihren Derivaten wie Acrylnitril oder Acrylamid und von Dienen wie Butadien, Chloropren oder Isopren und Vinylchlorid. Durch die Art der Verknüpfung kommt es praktisch nicht zu unerwünschten Latex-Latex-Verknüpfungen, sondern bei der unspezifischen Reaktion der Azidogruppe tritt nur die kovalente Verknüpfung von Latex und Protein auf und in geringem Umfang eine Verknüpfung von Protein mit Protein.

Die an den agglutinierbaren Teilchen gebundene Substanz und die Komponente K sind miteinander bindefähig. Alle spezifisch miteinander bindenden Paare sind hier geeignet. Bevorzugt wird als Komponente K und damit bindefähige Substanz Streptavidin bzw. Avidin und Biotin eingesetzt.

Bei Durchführung des Verfahrens, d.h. Inkubation der Probelösung mit dem Konjugat und den agglutinierbaren Teilchen, bindet einerseits die nachzuweisende Substanz an das Fab'-Fragment des Konjugats und andererseits die Komponente K des Konjugats an die agglutinierbaren Teilchen. Nur durch diese Bindungen kann eine Agglutination ausgelöst werden. Da das Konjugat bezüglich der Bindung an die nachzuweisende Substanz und bezüglich der Bindung an die agglutinierbaren Teilchen monovalent ist, kann eine Agglutination durch die Rezeptoren an sich nicht erfolgen. Je mehr nachzuweisende Substanz in der Probelösung vorhanden ist, eine desto stärkere Agglutination tritt auf. Das Maß der Agglutination, die nach bekannten Verfahren nachgewiesen werden kann, ist daher ein direktes Maß für den Gehalt an nachzuweisender Substanz.

Besonders geeignet ist das erfindungsgemäße Verfahren für Ausführungsformen, wie sie in EP-A 349 988 oder EP-A 356 964 beschrieben sind. Sie beschreiben homogene Immunoassays, bei denen eine Probelösung, die die nachzuweisende Substanz enthält, mit mindestens zwei Rezeptoren inkubiert wird, von denen einer ein Konjugat aus einem Partner eines spezifisch bindenden Paares und einer mit der zu bestimmenden Substanz spezifisch bindenden Komponente ist und der andere Rezeptor mindestens zwei Bindungsstellen für den spezifisch bindenden Partner aufweist. Durch Inkubation der Probelösung mit den beiden Rezeptoren kommt es zur Bindung des Konjugates an die nachzuweisende Substanz und an den anderen Rezeptor, wodurch eine Agglutination bewirkt wird. Agglutinieren können nur Komplexe, in denen die nachzuweisende Substanz und beide Rezeptoren gebunden sind. Um die Genauigkeit zu erhöhen, ist es bevorzugt, ein Konjugat mit bezüglich der Bindung monovalenten Anteilen zu verwenden.

Die Erfindung wird durch die folgenden Beispiele erläutert:

**Beispiel 1**

Herstellung von Streptavidin-Latex

Streptavidin (Boehringer Mannheim) wurde zu einer Konzentration von 10 mg/ml in 50 mM $K_2HPO_4$, pH 8,0 gelöst. Anschließend wurde im Dunkeln eine Lösung von N-Hydroxysuccinimidyl-4-azidobenzoat (HSAB) in DMSO zugegeben, so daß ein molares Verhältnis von 10:1 (HSAB:Streptavidin) resultierte. Bei 25°C wurde zwei Stunden unter Lichtausschluß inkubiert. Anschließend wurde überschüssiges HSAB durch Dialyse im Dunkeln abgetrennt.

Zur Beschichtung von Latices mit Streptavidin wurde eine 1%ige Suspension an Polystyrol-Partikel (Dow < 100 nm) unter Lichtausschluß mit dem aktivierten Streptavidin (1 mg/ml, Herstellung siehe oben) über Nacht inkubiert. Anschließend wurden die Partikel mit UV-Licht (340 nm) bestrahlt. Die Streptavidin-Latices wurden zweimal mit 50 mM Glycin, pH 7,5 gewaschen und danach 16 Stunden mit einer Nachbeladelösung (0,1 % RSA) inkubiert. Nach einem weiteren Waschschritt mit 50 mM Glycin, pH 7,5 wurden die Streptavidin-Latices in Glycin-Puffer bei 4°C gelagert.

**Beispiel 2**

Herstellung von Fab'-Biotin-Konjugaten

Monoklonale Antikörper der Immunglobulinklasse G I und G IIa, die in üblicher Weise durch Immunisierung von Mäusen gewonnen wurden, wurden nach dem im folgenden beschriebenen Verfahren zu Fab'-Biotin-Konjugaten umgesetzt:

50 mg an Maus-IgG wurden in 100 mM Natriumcitrat, pH 3,6 zu einer Proteinkonzentration von 10 mg/ml gelöst. Zu der auf 37°C erwärmten Lösung wurde eine Pepsinlösung (1 mg/ml in 100 mM Natriumcitrat, pH 3,6) im Verhältnis 1:1000 gegeben. Nach 2,5 Stunden unter Rühren bei 37°C wurde der pH-Wert der Reaktionsmischung durch Zugabe von Tris auf 8,3 eingestellt. Anschließend wurde gegen 20 mM Tris/HCl, pH 7,8 dialysiert. Die Abtrennung von unverdautem IgG und Fc-Fragmenten vom $F(ab')_2$ erfolgte durch

Chromatographie an DEAE-Sepharose® fast flow und Sephacryl® S-200. Die F(ab')$_2$-Fragmente wurden gesammelt und lyophilisiert.

10 mg F(ab')$_2$ wurden in 100 mM $KH_2PO_4$, 1 mM EDTA, pH 6,3 zu einer Konzentration von 10 mg/ml gelöst. Anschließend wurden 20 $\mu$l einer Lösung von 500 mM Natriumarsenit, 1 mM EDTA, 100 mM $KH_2PO_4$, pH 6,8 sowie 25 $\mu$l einer Lösung aus 200 mM Mercaptoethylamin, 1 mM EDTA, 100 mM $KH_2PO_4$, pH 6,8 zugesetzt. Bei 37°C wurde 2 Stunden unter Rühren inkubiert. Anschließend wurde der Fab'-Pool durch Chromatographie an einer Sephadex® G-25-Säule von den niedermolekularen Bestandteilen abgetrennt.

Für die Biotinylierung wurden Maleimidoethylamin-Biotin und Maleimidobutyryllysin-Biotin jeweils zu 1 mg/ml in DMSO gelöst. In getrennten Ansätzen wurde zu einer Lösung der Fab'-Fragmente Maleimidoethylamin-Biotin bzw. Maleimidobutyryllysin-Biotin so zugesetzt, daß ein molares Verhältnis von 10:1 (Biotinderivat:Fab') resultierte. Nach einer Stunde Inkubation bei 25°C wurde zunächst Cystein ad 1 mM und nach weiteren 30 Minuten Jodacetamid ad 5 mM zugesetzt. Nach einer weiteren Inkubation von 30 Minuten wurde gegen 2 mM $KH_2PO_4$, pH 7,5 dialysiert. Die erhaltenen Fab'-Biotin-Konjugate wurden bei -20°C gelagert.

**Beispiel 3**

Es wurde untersucht, inwieweit die unspezifische Agglutination durch Verwendung der erfindungsgemäßen Konjugate reduziert wird, in einem Test unter Verwendung von mit Streptavidin beschichteten Latex-Partikeln und Konjugaten aus Anti-TSH-Antikörperfragmentenund Biotin.

Es wurde ein monoklonaler Anti-TSH-Antikörper der Immunglobulinklasse G I (ECACC 87122202) verwendet. Zum Vergleich erfolgte eine Biotinylierung eines kompletten Antikörpers über die Aminogruppen der Antikörper gemäß JACS 100 (1978), 3585-3590 durch Umsetzung mit N-Hydroxysuccinimid-X-Biotin im Verhältnis 7:1 (Biotin:IgG).

Das erfindungsgemäße Fab'-Biotin-Konjugat aus diesem Antikörper wurde unter Verwendung von Maleimidobutyryllysin-Biotin hergestellt, wie im Beispiel 2 beschrieben.

Zur Bestimmung der Agglutination wurden 60 $\mu$l 1 %ige Streptavidin-Latex (Herstellung siehe Beispiel 1), 800 $\mu$l 100 mM Tris/HCl, pH 8,0 und 20 $\mu$l Anti-TSH-IgG-Biotin-Konjugat (Vergleich) bzw. 20 $\mu$l Anti-TSH-Fab'-Biotin-Konjugat (erfindungsgemäß) in Küvetten eines Perkin-Elmer Photometers pipettiert. Die Proteinkonzentration der Konjugate betrug jeweils 10 $\mu$g/ml.

Die Extinktionszunahme bei 405 nm wurde zwischen t = 1 min und t = 10 min gemessen.

Das Ergebnis ist in Tabelle 1 dargestellt. Es zeigte sich, daß die unspezifische Agglutination unter Verwendung des erfindungsgemäßen Fab'-Biotin-Konjugates auf weniger als 1/5 des Referenzwertes (IgG-Biotin-Konjugat) reduziert werden konnte.

## Tabelle  1

| Antikörper-Konjugat | Extinktions-Zunahme (mE) |
| --- | --- |
| IgG-Biotin (Vergleich) | 663 |
| Fab'-Biotin (erfindungsgemäß) | 119 |

**Beispiel 4**

Die unspezifische Agglutination von erfindungsgemäß hergestellten Konjugaten wurde wie in Beispiel 3 beschrieben untersucht.

Es wurden zwei verschiedene monoklonale Anti-Myoglobin-Antikörper, die beide der Immunglobulinklasse IgG I angehören, wie in Beispiel 2 beschrieben zu Fab'-Biotin-Konjugaten umgesetzt. Zur Untersuchung des Einflusses der Spacerlänge auf die unspezifische Agglutination wurden beide Fab'-Fragmente mit Biotin unter Verwendung von Maleimidoethylamin (MEA) und Maleimidobutytyllysin (MBL) als Spacer umgesetzt.

Die Ergebnisse sind in Tabelle 2 zusammengefaßt. Es wird deutlich, daß durch Verwendung des kürzeren MEA-Spacers die unspezifische Agglutination noch weiter reduziert werden kann.

Tabelle 2

| Antikörper-Konjugat (Fab'-Biotin) | Extinktions-Zunahme (mE) | |
|---|---|---|
| | MBL | MEA |
| Anti-Myoglobin-AK 1 | 267 | 88 |
| Anti-Myoglobin-AK 2 | 228 | 72 |

## Beispiel 5

Zwei verschiedene monoklonale Anti-AFP-Antikörper, von denen der eine der Immunglobulinklasse G I (Mab LJ738) und der andere der Klasse G IIa (Mab Tull, ECACC 87041002) angehört, wurden wie in Beispiel 2 beschrieben zu Fab'-Biotin-Konjugaten umgesetzt. Die beiden Fab'-Fragmente wurden dabei mit Biotin unter Verwendung von MEA bzw. MBL als Spacer biotinyliert.

Zur Bestimmung der unspezifischen Agglutination wurden jeweils 20 $\mu$l der verschiedenen Konjugate (10 $\mu$g/ml), 800 $\mu$l 100 mM Tris/HCl, 2 % PEG 40000, 2 % Pluronic® F68, pH 7,0 und 20 $\mu$l 1 %ige Streptavidin-Latex (Herstellung siehe Beispiel 1) in die Küvetten eines Perkin-Elmer-Photometers pipettiert. Die Extinktionszunahme wurde bei 405 nm zwischen t = 1 min und t = 10 min gemessen.

Die Ergebnisse sind in Tabelle 3 dargestellt.

Wie schon für die anderen monoklonalen Antikörper der Immunglobulinklasse I gemessen (vgl. Beispiele 3 und 4) wurde auch mit dem Anti-AFP-Fab'-Biotin aus IgG I eine niedrige unspezifische Agglutination erhalten, die sich durch Verwendung des kürzeren MEA-Spacers noch weiter unterdrücken ließ.

Das Anti-AFP-Fab'-Biotin-Konjugat aus dem IgG IIa dagegen ergab mit dem MBL-Spacer relativ hohe unspezifische Agglutination. Durch Verwendung des kürzeren MEA-Spacers konnten jedoch vorteilhafte Ergebnisse erzielt werden.

Tabelle 3

| Antikörper-Konjugat (Fab'-Biotin) | Extinktions-Zunahme (mE) | |
|---|---|---|
| | MBL | MEA |
| Anti-AFP-Mab-LJ738 (IgG I) | 42 | 3 |
| Anti-AFP-Mab-Tu11 (IgG IIa) | 434 | 212 |

## Beispiel 6

Mit den Konjugaten Mab-Tu11-Fab'-MEA-Biotin und Mab-LJ738-Fab'-MBL-Biotin wurde ein homogener Immunoassay auf Streptavidin-Latex-Basis durchgeführt:

50 $\mu$l einer Lösung der beiden Konjugate (10 $\mu$g/ml, Verhältnis der Antikörper-Konjugate von 1:1), 800 $\mu$l 100 mM Tris/HCl, 2 % PEG 40000, 2 % Pluronic® F68, pH 7,0 und 20 $\mu$l 1 %ige Streptavidin-Latex sowie jeweils 50 $\mu$l eines AFP-Standards (0, 50, 100, 1000 und 2000 ng/ml) wurden in Küvetten pipettiert. Die Zunahme der Agglutination wurde im Perkin-Elmer-Photometer bei 405 nm zwischen t = 1 min und t = 10 min gemessen.

EP 0 464 554 B1

Das Ergebnis des Immunoassays ist in Tabelle 4 dargestellt.

Tabelle 4

| | Standard Konzentration (ng/ml) | | | | | |
|---|---|---|---|---|---|---|
| | 0 | 50 | 100 | 500 | 1000 | 2000 |
| Extinktion (Mittelwert) | 44 | 51 | 60 | 151 | 299 | 547 |
| VK (%) | 17,6 | 12,8 | 12,2 | 8,4 | 4,5 | 1,9 |
| Std. 0 + 3s Bereich | 67 | - | - | - | - | - |

**Patentansprüche**
**Patentansprüche für folgende Vertragsstaaten : AT, BE, CH, DE, DK, FR, GB, GR, IT, LI, LU, NL, SE**

1. Verfahren zur Durchführung eines homogenen Immunoassays nach dem Agglutinationsprinzip unter Verwendung eines Konjugats aus einem Fab'-Fragment und einer Komponente K,
**dadurch gekennzeichnet,**
daß man ein Konjugat verwendet, welches erhalten wird indem man ein $F(ab')_2$-Fragment eines Antikörpers der Immunglobulinklasse G reduzierenden Bedingungen aussetzt und anschließend mit der Komponente K, die entweder eine zur Bindung geeignete funktionelle Gruppe aufweist oder durch Einführung einer funktionellen Gruppe in geeigneter Weise derivatisiert wurde, umsetzt, wobei die Komponente K über die funktionelle Gruppe an die bei der Reduktion entstandene freie SH-Gruppe des Fab'-Fragmentes gebunden wird und agglutinierbare Teilchen verwendet, welche eine mit K bindefähige Substanz tragen.

2. Verfahren nach Anspruch 1
**dadurch gekennzeichnet,**
daß man als Komponente K ein Hapten verwendet.

3. Verfahren nach Anspruch 2,
**dadurch gekennzeichnet,**
daß man als Hapten Biotin verwendet.

4. Verfahren nach einem der vorhergehenden Ansprüche,
**dadurch gekennzeichnet,**
daß man das Fab'-Fragment und die Komponente K über einen Spacer verbindet.

5. Verfahren nach einem der vorhergehenden Ansprüche,
**dadurch gekennzeichnet,**
daß man ein Fab'-Fragment eines Antikörpers der IgG-Subklasse I verwendet und einen Spacer mit einer Kettenlänge von 4 bis 16 Atomen einsetzt.

7

6. Verfahren nach einem der Ansprüche 1 bis 4,
**dadurch gekennzeichnet,**
daß man Fab'-Fragmente eines IgG-Antikörpers der Subklasse II verwendet und einen Spacer einsetzt mit einer Länge von 4 bis 10 C-Atomen.

7. Verfahren nach einem der vorhergehenden Ansprüche,
**dadurch gekennzeichnet,**
daß als agglutinierbare Teilchen Latexteilchen eingesetzt werden, an die über eine bifunktionelle Verbindung eine mit der Komponente K bindefähige Substanz gebunden ist.

8. Verfahren nach Anspruch 7,
**dadurch gekennzeichnet,**
daß man das agglutinierbare Teilchen herstellt, indem man eine mit der Komponente K bindefähige Substanz mit einer bifunktionellen Verbindung, die eine N-Hydroxysuccinimidester-Gruppe und eine Arylazidgruppe aufweist, unter schwach alkalischen Bedingungen in wäßriger Lösung umsetzt, überschüssige bifunktionelle Verbindung danach abtrennt, einen Latex zu dieser Lösung zufügt und dann mit Photonen bestrahlt und dadurch die Bindung an den Latex bewirkt.

9. Konjugate, bestehend aus einem Fab'-Fragment eines IgG-Antikörpers und einer Komponente K, welche erhalten werden, indem man ein $F(ab')_2$-Fragment eines Antikörpers der Immunglobulinklasse G reduzierenden Bedingungen aussetzt und anschließend mit der Komponente K, die entweder eine zur Bindung geeignete funktionelle Gruppe aufweist oder durch Einführung einer funktionellen Gruppe in geeigneter Weise derivatisiert wurde, umsetzt, wobei die Komponente K über die funktionelle Gruppe an die bei der Reduktion entstandene freie SH-Gruppe des Fab'-Fragmentes gebunden wird.

10. Konjugate nach Anspruch 9,
**dadurch gekennzeichnet,**
daß die Bindung zwischen dem Fab'-Fragment und der Komponente K direkt zwischen SH-Gruppen des Fab'-Fragments und der funktionellen Gruppe der Komponente K oder über einen Spacer erfolgt.

11. Konjugate nach Anspruch 10,
**dadurch gekennzeichnet,**
daß der Spacer eine bifunktionelle Verbindung ist, wobei eine funktionelle Gruppe mit einer SH-Gruppe des Fab'-Fragments bindefähig ist, und die zweite funktionelle Gruppe, die gleich oder verschieden sein kann, mit der Komponente K eine kovalente Bindung eingehen kann.

12. Konjugate nach Anspruch 10 oder 11,
**dadurch gekennzeichnet,**
daß das Fab'-Fragment von einem Antikörper der IgG-Subklasse I stammt und der Spacer eine Kettenlänge von 4 bis 16 Atomen aufweist.

13. Konjugate nach Anspruch 10 oder 11,
**dadurch gekennzeichnet,**
daß das Fab'-Fragment von einem Antikörper der IgG-Subklasse II stammt und der Spacer eine Kettenlänge von 4 bis 10 C-Atomen aufweist.

14. Konjugate nach einem der Ansprüche 9-13,
**dadurch gekennzeichnet,**
daß die Komponente K ein Hapten ist.

15. Konjugate nach einem der Ansprüche 9-14,
**dadurch gekennzeichnet,**
daß die Komponente K Biotin ist.

16. Verwendung von Konjugaten nach einem der Ansprüche 9-15 in einem homogenen Immunoassay, bei dem keine Trennung von gebundener und ungebundener Markierung erfolgt.

**Patentansprüche für folgenden Vertragsstaat : ES**

1. Verfahren zur Durchführung eines homogenen Immunoassays nach dem Agglutinationsprinzip unter Verwendung eines Konjugats aus einem Fab'-Fragment und einer Komponente K,
**dadurch gekennzeichnet,**
daß man ein Konjugat verwendet, welches erhalten wird indem man ein $F(ab')_2$-Fragment eines Antikörpers der Immunglobulinklasse G reduzierenden Bedingungen aussetzt und anschließend mit der Komponente K, die entweder eine zur Bindung geeignete funktionelle Gruppe aufweist oder durch Einführung einer funktionellen Gruppe in geeigneter Weise derivatisiert wurde, umsetzt, wobei die Komponente K über die funktionelle Gruppe an die bei der Reduktion entstandene freie SH-Gruppe des Fab'-Fragmentes gebunden wird und agglutinierbare Teilchen verwendet, welche eine mit K bindefähige Substanz tragen.

2. Verfahren nach Anspruch 1
**dadurch gekennzeichnet,**
daß man als Komponente K ein Hapten verwendet.

3. Verfahren nach Anspruch 2,
**dadurch gekennzeichnet,**
daß man als Hapten Biotin verwendet.

4. Verfahren nach einem der vorhergehenden Ansprüche,
**dadurch gekennzeichnet,**
daß man das Fab'-Fragment und die Komponente K über einen Spacer verbindet.

5. Verfahren nach einem der vorhergehenden Ansprüche,
**dadurch gekennzeichnet,**
daß man ein Fab'-Fragment eines Antikörpers der IgG-Subklasse I verwendet und einen Spacer mit einer Kettenlänge von 4 bis 16 Atomen einsetzt.

6. Verfahren nach einem der Ansprüche 1 bis 4,
**dadurch gekennzeichnet,**
daß man Fab'-Fragmente eines IgG-Antikörpers der Subklasse II verwendet und einen Spacer einsetzt mit einer Länge von 4 bis 10 C-Atomen.

7. Verfahren nach einem der vorhergehenden Ansprüche,
**dadurch gekennzeichnet,**
daß als agglutinierbare Teilchen Latexteilchen eingesetzt werden, an die über eine bifunktionelle Verbindung eine mit der Komponente K bindefähige Substanz gebunden ist.

8. Verfahren nach Anspruch 7,
**dadurch gekennzeichnet,**
daß man das agglutinierbare Teilchen herstellt, indem man eine mit der Komponente K bindefähige Substanz mit einer bifunktionellen Verbindung, die eine N-Hydroxysuccinimidester-Gruppe und eine Arylazidgruppe aufweist, unter schwach alkalischen Bedingungen in wäßriger Lösung umsetzt, überschüssige bifunktionelle Verbindung danach abtrennt, einen Latex zu dieser Lösung zufügt und dann mit Photonen bestrahlt und dadurch die Bindung an den Latex bewirkt.

9. Verfahren zur Herstellung von Konjugaten, bestehend aus einem Fab'-Fragment eines IgG-Antikörpers und einer Komponente K,
**dadurch gekennzeichnet, daß** man ein $F(ab')_2$-Fragment eines Antikörpers der Immunglobulinklasse G reduzierenden Bedingungen aussetzt und anschließend mit der Komponente K, die entweder eine zur Bindung geeignete funktionelle Gruppe aufweist oder durch Einführung einer funktionellen Gruppe in geeigneter Weise derivatisiert wurde, umsetzt, wobei die Komponente K über die funktionelle Gruppe an die bei der Reduktion entstandene freie SH-Gruppe des Fab'-Fragmentes gebunden wird.

10. Verfahren nach Anspruch 9,
**dadurch gekennzeichnet,**

daß die Herstellung der Bindung zwischen dem Fab'-Fragment und der Komponente K direkt zwischen SH-Gruppen des Fab'-Fragments und der funktionellen Gruppe der Komponente K oder über einen Spacer erfolgt.

**11.** Verfahren nach Anspruch 10,
**dadurch gekennzeichnet,**
daß der Spacer eine bifunktionelle Verbindung ist, wobei eine funktionelle Gruppe mit einer SH-Gruppe des Fab'-Fragments bindefähig ist, und die zweite funktionelle Gruppe, die gleich oder verschieden sein kann, mit der Komponente K eine kovalente Bindung eingehen kann.

**12.** Verfahren nach Anspruch 10 oder 11,
**dadurch gekennzeichnet,**
daß das Fab'-Fragment von einem Antikörper der IgG-Subklasse I stammt und der Spacer eine Kettenlänge von 4 bis 16 Atomen aufweist.

**13.** Verfahren nach Anspruch 10 oder 11,
**dadurch gekennzeichnet,**
daß das Fab'-Fragment von einem Antikörper der IgG-Subklasse II stammt und der Spacer eine Kettenlänge von 4 bis 10 C-Atomen aufweist.

**14.** Verfahren nach einem der Ansprüche 9-13,
**dadurch gekennzeichnet,**
daß die Komponente K ein Hapten ist.

**15.** Verfahren nach einem der Ansprüche 9-14,
**dadurch gekennzeichnet,**
daß die Komponente K Biotin ist.

**16.** Verwendung von Konjugaten nach einem der Ansprüche 9-15 in einem homogenen Immunoassay, bei dem keine Trennung von gebundener und ungebundener Markierung erfolgt.

**Claims**
**Claims for the following Contracting States : AT, BE, CH, DE, DK, FR, GB, GR, IT, LI, LU, NL, SE**

**1.** Method for carrying out a homogeneous immunoassay based on agglutination in which a conjugate of a Fab' fragment and a component K is used,
**wherein**
a conjugate is used which is obtained by subjecting a $F(ab')_2$ fragment of an antibody of the immunoglobulin G class to reducing conditions and subsequently reacting it with the component K which either has a functional group suitable for the binding or was derivatized in a suitable manner by introduction of a functional group whereby the component K is bound via the functional group to the free SH group of the Fab' fragment formed during the reduction and agglutinable particles are used which carry a substance capable of binding to K.

**2.** Method as claimed in claim 1,
**wherein**
a hapten is used as the component K.

**3.** Method as claimed in claim 2,
**wherein**
biotin is used as the hapten.

**4.** Method as claimed in one of the previous claims,
**wherein**
the Fab' fragment and the component K are linked via a spacer.

**5.** Method as claimed in one of the previous claims,
**wherein**

a Fab' fragment of an antibody of the IgG subclass I is used and a spacer is employed with a chain length of 4 to 16 atoms.

6. Method as claimed in one of the claims 1 to 4,

    **wherein**

    Fab' fragments of an IgG antibody of the subclass II are used and a spacer is employed with a length of 4 to 10 C-atoms.

7. Method as claimed in one of the previous claims,

    **wherein**

    latex particles are used as the agglutinable particles to which a substance capable of binding to the component K is bound via a bifunctional compound.

8. Method as claimed in claim 7,

    **wherein**

    the agglutinable particle is produced by reacting a substance capable of binding to the component K with a bifunctional compound which has a N-hydroxysuccinimide ester group and an arylazide group under weak alkaline conditions in an aqueous solution, excess bifunctional compound is subsequently removed, a latex is added to this solution and then it is iradiated with photons which results in binding to the latex.

9. Conjugates composed of a Fab' fragment of an IgG antibody and a component K which are obtained by subjecting a $F(ab')_2$ fragment of an antibody of the immunoglobulin G class to reducing conditions and subsequently reacting it with the component K which either has a functional group suitable for the binding or was derivatized in a suitable manner by introduction of a functional group whereby the component K is bound via the functional group to the free SH group of the Fab' fragment formed during the reduction.

10. Conjugates as claimed in claim 9,

    **wherein**

    the binding between the Fab' fragment and the component K either takes place directly between SH groups of the Fab' fragment and a functional group of the component K or via a spacer.

11. Conjugates as claimed in claim 10,

    **wherein**

    the spacer is a bifunctional compound in which a functional group is capable of binding to a SH group of the Fab' fragment and the second functional group which can be the same or different can covalently bind to the component K.

12. Conjugates as claimed in claim 10 or 11,

    **wherein**

    the Fab' fragment is derived from an antibody of the IgG subclass I and the spacer has a chain length of 4 to 10 C atoms.

13. Conjugates as claimed in claim 10 or 11,

    **wherein**

    the Fab' fragment is derived from an antibody of the IgG subclass II and the spacer has a chain length of 4 to 10 C atoms.

14. Conjugates as claimed in one of the claims 9-13,

    **wherein**

    the component K is a hapten.

15. Conjugates as claimed in one of the claims 9-14,

    **wherein**

    the component K is biotin.

16. Use of conjugates as claimed in one of the claims 9-15 in a homogeneous immunoassay in which there is no separation of bound and unbound label.

**Claims for the following Contracting State : ES**

1. Method for carrying out a homogeneous immunoassay based on agglutination in which a conjugate of a Fab' fragment and a component K is used,
   **wherein**
   a conjugate is used which is obtained by subjecting a F(ab')$_2$ fragment of an antibody of the immunoglobulin G class to reducing conditions and subsequently reacting it with the component K which either has a functional group suitable for the binding or was derivatized in a suitable manner by introduction of a functional group whereby the component K is bound via the functional group to the free SH group of the Fab' fragment formed during the reduction and agglutinable particles are used which carry a substance capable of binding to K.

2. Method as claimed in claim 1,
   **wherein**
   a hapten is used as the component K.

3. Method as claimed in claim 2,
   **wherein**
   biotin is used as the hapten.

4. Method as claimed in one of the previous claims,
   **wherein**
   the Fab' fragment and the component K are linked via a spacer.

5. Method as claimed in one of the previous claims,
   **wherein**
   a Fab' fragment of an antibody of the IgG subclass I is used and a spacer is employed with a chain length of 4 to 16 atoms.

6. Method as claimed in one of the claims 1 to 4,
   **wherein**
   Fab' fragments of an IgG antibody of the subclass II are used and a spacer is employed with a length of 4 to 10 C-atoms.

7. Method as claimed in one of the previous claims,
   **wherein**
   latex particles are used as the agglutinable particles to which a substance capable of binding to the component K is bound via a bifunctional compound.

8. Method as claimed in claim 7,
   **wherein**
   the agglutinable particle is produced by reacting a substance capable of binding to the component K with a bifunctional compound which has a N-hydroxysuccinimide ester group and an arylazide group under weak alkaline conditions in an aqueous solution, excess bifunctional compound is subsequently removed, a latex is added to this solution and then it is iradiated with photons which results in binding to the latex.

9. Method for the production of conjugates composed of a Fab' fragment of an IgG antibody and a component K,
   **wherein**
   a F(ab')$_2$ fragment of an antibody of the immunoglobulin G class is subjected to reducing conditions and subsequently reacted with the component K which either has a functional group suitable for the binding or was derivatized in a suitable manner by introduction of a functional group whereby the component K is bound via the functional group to the free SH group of the Fab' fragment formed during the reduction.

12

**10.** Method as claimed in claim 9,
**wherein**
the binding between the Fab' fragment and the component K is either effected directly between SH groups of the Fab' fragment and a functional group of the component K or via a spacer.

**11.** Method as claimed in claim 10,
**wherein**
the spacer is a bifunctional compound in which a functional group is capable of binding to a SH group of the Fab' fragment and the second functional group which can be the same or different can covalently bind to the component K.

**12.** Method as claimed in claim 10 or 11,
**wherein**
the Fab' fragment is derived from an antibody of the IgG subclass I and the spacer has a chain length of 4 to 16 C atoms.

**13.** Method as claimed in claim 10 or 11,
**wherein**
the Fab' fragment is derived from an antibody of the IgG subclass II and the spacer has a chain length of 4 to 10 C atoms.

**14.** Method as claimed in one of the claims 9-13,
**wherein**
the component K is a hapten.

**15.** Method as claimed in one of the claims 9-14,
**wherein**
the component K is biotin.

**16.** Use of conjugates as claimed in one of the claims 9-15 in a homogeneous immunoassay in which there is no separation of bound and unbound label.

**Revendications**
**Revendications pour les Etats contractants suivants : AT, BE, CH, DE, DK, FR, GB, GR, IT, LI, LU, NL, SE**

**1.** Procédé pour réaliser un immunodosage homogène selon le principe d'agglutination au moyen d'un conjugué d'un fragment Fab' et d'un constituant K, caractérisé en ce que l'on utilise un conjugué qui est obtenu en exposant à des conditions réductrices un fragment $F(ab')_2$ d'un anticorps de la classe G des immunoglobulines puis en le faisant réagir avec le constituant K qui présente un groupe fonctionnel convenant pour la liaison ou qui a été transformé en dérivé de manière appropriée par introduction d'un groupe fonctionnel, le constituant K se liant par l'intermédiaire du groupe fonctionnel au groupe SH libre, formé lors de la réduction, du fragment Fab' et l'on utilise des particules agglutinables qui portent une substance capable de se lier à K.

**2.** Procédé selon la revendication 1, caractérisé en ce que l'on utilise un haptène comme constituant K.

**3.** Procédé selon la revendication 2, caractérisé en ce que l'on utilise la biotine comme haptène.

**4.** Procédé selon l'une des revendications précédentes, caractérisé en ce qu'on lie le fragment Fab' et le constituant K par l'intermédiaire d'un espaceur.

**5.** Procédé selon l'une des revendications précédentes, caractérisé en ce que l'on utilise un fragment Fab' d'un anticorps de la sous-classe I des IgG et l'on emploie un espaceur d'une longueur de chaîne de 4 à 16 atomes.

**6.** Procédé selon l'une des revendications 1 à 4, caractérisé en ce que l'on utilise des fragments Fab' d'un anticorps IgG de la sous-classe II et l'on emploie un espaceur d'une longueur de 4 à 10 atomes

13

de C.

**7.** Procédé selon l'une des revendications précédentes, caractérisé en ce que l'on utilise comme particules agglutinables des particules de latex auxquelles est liée par l'intermédiaire d'un composé bifonctionnel une substance capable de se lier au constituant K.

**8.** Procédé selon la revendication 7, caractérisé en ce que l'on prépare les particules agglutinables en faisant réagir dans des conditions faiblement alcalines en solution aqueuse une substance capable de se lier au constituant K avec un composé bifonctionnel qui présente un groupe ester de N-hydroxysuccinimide et un groupe arylazide, puis on sépare le composé bifonctionnel en excès, on ajoute un latex à cette solution puis on irradie avec des photons et on forme ainsi la liaison au latex.

**9.** Conjugués consistant en un fragment Fab' d'un anticorps IgG et en un constituant K qui sont obtenus en exposant un fragment F(ab')$_2$ d'un anticorps de la classe G des immunoglobulines à des conditions réductrices puis en le faisant réagir avec le constituant K qui présente un groupe fonctionnel convenant pour la liaison ou qui a été transformé en dérivé de manière appropriée par introduction d'un groupe fonctionnel, le constituant K se liant par l'intermédiaire du groupe fonctionnel au groupe SH libre, formé lors de la réduction, du fragment Fab'.

**10.** Conjugués selon la revendication 9, caractérisés en ce que la liaison entre le fragment Fab' et le constituant K se produit directement entre des groupes SH du fragment Fab' et le groupe fonctionnel du constituant K ou par l'intermédiaire d'un espaceur.

**11.** Conjugués selon la revendication 10, caractérisés en ce que l'espaceur est un composé bifonctionnel, un groupe fonctionnel étant capable de se lier à un groupe SH du fragment Fab' et le second groupe fonctionnel, qui peut être identique ou différent, pouvant engager une liaison covalente avec le constituant K.

**12.** Conjugués selon la revendication 10 ou 11, caractérisés en ce que le fragment Fab' provient d'un anticorps de la sous-classe I des IgG et l'espaceur présente une longueur de chaîne de 4 à 16 atomes.

**13.** Conjugués selon la revendication 10 ou 11, caractérisés en ce que le fragment Fab' provient d'un anticorps de la sous-classe II des IgG et l'espaceur présente une longueur de chaîne de 4 à 10 atomes de C.

**14.** Conjugués selon l'une des revendications 9 à 13, caractérisés en ce que le constituant K est un haptène.

**15.** Conjugués selon l'une des revendications 9 à 14, caractérisés en ce que le constituant K est la biotine.

**16.** Utilisation de conjugués selon l'une des revendications 9 à 15 dans un immunodosage homogène dans lequel aucune séparation n'a lieu entre le marqueur lié et le marqueur non lié.

**Revendications pour l'Etat contractant suivant : ES**

**1.** Procédé pour réaliser un immunodosage homogène selon le principe d'agglutination au moyen d'un conjugué d'un fragment Fab' et d'un constituant K, caractérisé en ce que l'on utilise un conjugué qui est obtenu en exposant à des conditions réductrices un fragment F(ab')$_2$ d'un anticorps de la classe G des immunoglobulines puis en le faisant réagir avec le constituant K qui présente un groupe fonctionnel convenant pour la liaison ou qui a été transformé en dérivé de manière appropriée par introduction d'un groupe fonctionnel, le constituant K se liant par l'intermédiaire du groupe fonctionnel au groupe SH libre, formé lors de la réduction, du fragment Fab' et l'on utilise des particules agglutinables qui portent une substance capable de se lier à K.

**2.** Procédé selon la revendication 1, caractérisé en ce que l'on utilise un haptène comme constituant K.

**3.** Procédé selon la revendication 2, caractérisé en ce que l'on utilise la biotine comme haptène.

4. Procédé selon l'une des revendications précédentes, caractérisé en ce qu'on lie le fragment Fab' et le constituant K par l'intermédiaire d'un espaceur.

5. Procédé selon l'une des revendications précédentes, caractérisé en ce que l'on utilise un fragment Fab' d'un anticorps de la sous-classe I des IgG et l'on emploie un espaceur d'une longueur de chaîne de 4 à 16 atomes.

6. Procédé selon l'une des revendications 1 à 4, caractérisé en ce que l'on utilise des fragments Fab' d'un anticorps IgG de la sous-classe II et l'on emploie un espaceur d'une longueur de 4 à 10 atomes de C.

7. Procédé selon l'une des revendications précédentes, caractérisé en ce que l'on utilise comme particules agglutinables des particules de latex auxquelles est liée par l'intermédiaire d'un composé bifonctionnel une substance capable de se lier au constituant K.

8. Procédé selon la revendication 7, caractérisé en ce que l'on prépare les particules agglutinables en faisant réagir dans des conditions faiblement alcalines en solution aqueuse une substance capable de se lier au constituant K avec un composé bifonctionnel qui présente un groupe ester de N-hydroxysuccinimide et un groupe arylazide, puis on sépare le composé bifonctionnel en excès, on ajoute un latex à cette solution puis on irradie avec des photons et on forme ainsi la liaison au latex.

9. Procédé de préparation de conjugués consistant en un fragment Fab' d'un anticorps IgG et en un constituant K, caractérisé en ce que l'on expose un fragment $F(ab')_2$ d'un anticorps de la classe G des immunoglobulines à des conditions réductrices puis on le fait réagir avec le constituant K qui présente un groupe fonctionnel convenant pour la liaison ou qui a été transformé en dérivé de manière appropriée par introduction d'un groupe fonctionnel, le constituant K se liant par l'intermédiaire du groupe fonctionnel au groupe SH libre, formé lors de la réduction, du fragment Fab'.

10. Procédé selon la revendication 9, caractérisé en ce que la formation de la liaison entre le fragment Fab' et le constituant K se produit directement entre des groupes SH du fragment Fab' et le groupe fonctionnel du constituant K ou par l'intermédiaire d'un espaceur.

11. Procédé selon la revendication 10, caractérisé en ce que l'espaceur est un composé bifonctionnel, un groupe fonctionnel étant capable de se lier à un groupe SH du fragment Fab' et le second groupe fonctionnel, qui peut être identique ou différent, pouvant engager une liaison covalente avec le constituant K.

12. Procédé selon la revendication 10 ou 11, caractérisé en ce que le fragment Fab' provient d'un anticorps de la sous-classe I des IgG et l'espaceur présente une longueur de chaîne de 4 à 16 atomes.

13. Procédé selon la revendication 10 ou 11, caractérisé en ce que le fragment Fab' provient d'un anticorps de la sous-classe II des IgG et l'espaceur présente une longueur de chaîne de 4 à 10 atomes de C.

14. Procédé selon l'une des revendications 9 à 13, caractérisé en ce que le constituant K est un haptène.

15. Procédé selon l'une des revendications 9 à 14, caractérisé en ce que le constituant K est la biotine.

16. Utilisation de conjugués selon l'une des revendications 9 à 15 dans un immunodosage homogène dans lequel aucune séparation n'a lieu entre le marqueur lié et le marqueur non lié.